# FASCICULE DE BREVET EUROPEEN

(11) **EP 1 367 964 B1**
(45) Date de publication et mention de la délivrance du brevet: **25.07.2007**
(21) Numéro de dépôt: 02722339.5
(22) Date de dépôt: 12.03.2002
(51) Int. Cl.: A61F 2/16

(54) **INJECTEUR POUR LENTILLE INTRAOCULAIRE**
INJEKTOR FÜR EINE INTRAOKULARLINSE
INJECTOR FOR AN INTRAOCULAR LENS

(30) Priorité: 16.03.2001 FR 0103636
(43) Date de publication de la demande: 10.12.2003
(73) Titulaire: Laboratoire de Contactologie Appliquée - LCA, 28000 Chartres (FR)
(72) Inventeur: VINCENT, Patrice, F-28130 Mevoisins (FR)
(74) Mandataire: CAPRI
(86) Numéro de dépôt international: PCT/FR2002/000870
(87) Numéro de publication internationale: WO 2002/074202

(56) Documents cités:
- WO-A-96/13229
- WO-A-99/21514
- FR-A- 2 789 890
- US-A- 4 957 505

## Description

La présente invention concerne un injecteur pour lentille intraoculaire, et plus particulièrement un tel injecteur comportant un piston multibrins.

Un ensemble selon le préambule de la revendication 1 est connu du document FR-A-2 789 890.

De manière connue, une lentille intraoculaire est destinée à remplacer le cristallin d'un patient opéré de la cataracte. L'haptique de la lentille intraoculaire est la partie qui se situe à l'extérieur de l'optique et qui sert à maintenir la lentille en position à l'intérieur de l'oeil du patient. De manière habituelle, l'haptique inférieure est celle qui est introduite en premier, sachant que l'incision est le plus souvent située du côté frontal, le chirurgien étant durant l'intervention placé derrière la tête du patient couché. L'haptique supérieure qui est placée en dernier, reste la plus proche de l'incision.

Le concept de l'injecteur est apparu avec celui des lentilles intraoculaires réalisé en matériau souple, déformable mécaniquement. Ce développement répond à l'utilisation pour la chirurgie de la cataracte de petites incisions, dont la largeur est souvent limitée à environ 3 mm. L'embout de l'injecteur devrait pouvoir passer à travers cette incision sans qu'il soit besoin de l'élargir.

Il existe de nombreux modèles d'injecteurs pour lentille intraoculaire utilisant tous des pistons à un seul brin. Dans la plupart des cas, la lentille intraoculaire est pliée par un dispositif annexe tel qu'une cartouche à volet, un tiroir ou une nervure d'appui, de telle sorte que le rôle du piston se résume à la poussée finale à travers l'embout.

L'embout cylindrique de l'injecteur est de diamètre moyen interne nettement plus petit (1,5 à 3 mm) que le diamètre optique de la lentille intraoculaire (5 à 7 mm). L'idée de faire passer cette lentille dans une section se réduisant progressivement, donc d'allure plus ou moins conique a, déjà été divulguée dans des brevets antérieurs, et notamment dans le document WO 00/49974. Ce document divulgue un piston multibrins, lesdits brins se rapprochant progressivement pour finalement remplir la totalité de la section de l'injecteur. Ceci implique un risque de coincement de la lentille entre les brins dudit piston multibrins.

La présente invention a pour but de fournir un injecteur pour lentille intraoculaire qui ne reproduit pas les inconvénients susmentionnés.

La présente invention a notamment pour but de fournir un tel injecteur qui soit simple et peu coûteux à fabriquer et à réaliser.

Selon un mode de réalisation avantageux de la présente invention, l'injecteur prévoit de pousser la lentille intraoculaire de chaque côté en agissant vers deux points diamétralement opposés de son optique. Cette disposition a pour effet de conserver l'orientation de la lentille tout en limitant le frottement. Les bras latéraux qui sont en contact constant avec la paroi interne de la seringue empêchent la lentille d'y adhérer. La difficulté provient en effet du frottement de la lentille sur les parois qui peut conduire à la déstabiliser. Ainsi, si la lentille amorce un basculement il y a alors le risque qu'elle se coince entre le piston et la paroi. L'objet de l'invention, selon un mode de réalisation avantageux de celle-ci, qui associe au minimum deux brins latéraux poussant la lentille intraoculaire tout en la guidant, est de palier à cet inconvénient.

D'autre part, lorsqu'ils franchissent la partie conique de la seringue, les brins latéraux se rapprochent l'un de l'autre et contribuent au pliage de la lentille, en la chassant vers l'axe du système. L'haptique est alors simultanément resserrée par ses bords inférieurs (action du cône statique) et par ses bords supérieurs (action des brins latéraux).

La poussée des brins latéraux peut s'exercer soit directement sur l'haptique, soit par l'intermédiaire de l'haptique supérieure qui pourra alors se déformer. En cours de pliage, l'haptique supérieure peut être maintenue latéralement ou chassée vers l'axe. La forme de l'extrémité des brins latéraux peut donc être adaptée selon le dessin de la lentille et plus ou moins inclinée par rapport à la paroi de la seringue.

En fin de pliage, et afin d'éviter le pincement de l'haptique supérieure entre les brins et éventuellement la paroi, des espaces sont ménagés à l'extrémité des brins, soit au centre soit latéralement.

Bien entendu, l'invention ne s'oppose pas au fait que le chirurgien pourra ou non utiliser une solution viscoélastique aux propriétés lubrifiantes, afin d'améliorer ce processus.

Le présente brevet ne concernant que le système de poussée et de pliage de la lentille intraoculaire, il n'est fait aucune référence au mode de chargement de cette lentille dans le corps de seringue, ni au fait que le dispositif peut comporter des éléments réutilisables ou à usage unique. Dans les exemples de réalisation qui suivent, la lentille intraoculaire est considérée avoir été mise en place dans le système, sans avoir subie de déformation, en étant prête à être injectée.

La présente invention a donc pour objet un injecteur pour lentille intraoculaire comportant une optique, au moins une haptique inférieure, et au moins une haptique supérieure, ledit injecteur comprenant un corps de seringue comportant une partie principale, de préférence cylindrique, une partie d'éjection, de préférence cylindrique et de section transversale inférieure à celle de la partie principale, et une partie conique reliant ladite partie principale à ladite partie d'éjection, un piston étant adapté à se déplacer à l'intérieur dudit corps de seringue pour déplacer ladite lentille de la partie principale vers la partie d'éjection, puis pour éjecter ladite lentille hors de ladite partie d'éjection, ledit piston comportant au moins deux brins pouvant se rapprocher l'un de l'autre lorsque le piston coulisse dans ladite partie conique, caractérisé en ce que ledit piston, lorsqu'il atteint la partie d'éjection, comporte à son extrémité en contact avec la lentille un profil définissant au moins un espace adapté à recevoir ladite au moins une haptique supérieure de ladite lentille.

Avantageusement, en amont dudit espace, ledit piston remplit sensiblement toute la section transversale de ladite partie d'éjection.

Selon un premier mode de réalisation, ledit espace est central et est défini entre lesdits aux moins deux brins du piston.

Selon une première variante de réalisation, ledit piston est constitué de deux brins latéraux comportant à leurs extrémités en contact avec la lentille un évidement sur leurs côtés se faisant face, de sorte que lorsque lesdits deux brins sont sollicités l'un contre l'autre dans ladite partie d'éjection, lesdits évidements définissent un espace central.

Selon une seconde variante de réalisation, ledit piston est constitué de deux brins latéraux de même longueur et d'un brin central plus court, de sorte que lorsque lesdits brins latéraux sont sollicités contre ledit brin central dans ladite partie d'éjection, ledit brin central plus court définit un espace central entre les extrémités desdits brins latéraux.

Selon un autre mode de réalisation, ledit piston est constitué de deux brins latéraux de même longueur et d'un brin central plus long, de sorte que lorsque lesdits brins latéraux sont sollicités contre ledit brin central dans ladite partie d'éjection, ledit brin central plus long définit un espace latéral autour de l'extrémité plus longue dudit brin central.

Avantageusement, lesdits brins du piston sont adaptés à pousser la lentille dans le corps de seringue, la lentille glissant contre les parois dudit corps de seringue et étant déformée par ladite partie conique.

Selon un troisième mode de réalisation, ledit piston comporte deux brins latéraux et un brin central adapté à se déplacer par rapport auxdits brins latéraux entre une position de déformation et une position d'éjection.

Avantageusement, ladite lentille est disposée entre lesdits brins latéraux formant une pince, de telle sorte que ladite lentille ne glisse pas contre les parois dudit corps de seringue pendant sa déformation, ladite déformation étant réalisée par lesdits brins latéraux qui se rapprochent lorsque le piston coulisse dans ladite partie conique du corps de seringue.

Avantageusement, ledit brin central se déplace ensemble avec lesdits brins latéraux pendant la déformation de ladite lentille, jusqu'à une butée bloquant lesdits brins latéraux, ledit brin central se désolidarisant alors desdits brins latéraux pour éjecter ladite lentille déformée.

Avantageusement, ladite butée est formée au niveau de la jonction entre la partie conique et la partie d'éjection du corps de seringue.

Avantageusement, ledit brin central comporte à son extrémité en contact avec la lentille, un profil définissant un espace central ou latéral pour recevoir ladite au moins une haptique supérieure de ladite lentille.

Avantageusement, ledit brin central est relié auxdits brins latéraux par un mécanisme de liaison mécanique qui désolidarise automatiquement ledit brin central desdits brins latéraux lorsque ceux-ci atteignent ladite butée.

Avantageusement, en fin d'éjection de la lentille, ledit profil d'extrémité du piston, qui définit ledit espace, est disposé à l'extérieur du corps de seringue, en particulier à l'extérieur de ladite partie d'éjection.

Avantageusement, ladite partie principale du corps de seringue est adaptée à contenir la lentille non déformée.

Avantageusement, ladite partie principale du corps de seringue est aplatie en section transversale.

D'autres caractéristiques et avantages de la présente invention apparaîtront plus clairement au cours de la description détaillée suivante de quatre modes de réalisation de la présente invention, faite en référence aux dessins joints, donnés à titre d'exemples non limitatifs, et sur lesquels,
- les figures 1 à 3 représentent des vues schématiques en section transversale d'un premier mode de réalisation de la présente invention, respectivement en début, en cours et en fin de distribution de la lentille intraoculaire,
- les figures 4 à 6 représentent des vues similaires aux figures 1 à 3, représentant un second mode de réalisation de la présente invention,
- la figure 7 est une vue schématique en section transversale d'un troisième mode de réalisation de la présente invention, avant distribution de la lentille intraoculaire, et
- les figures 8 à 10 représentent des vues schématiques en section transversale d'un quatrième mode de réalisation de l'invention, respectivement avant, pendant et en fin de distribution de ladite lentille intraoculaire.

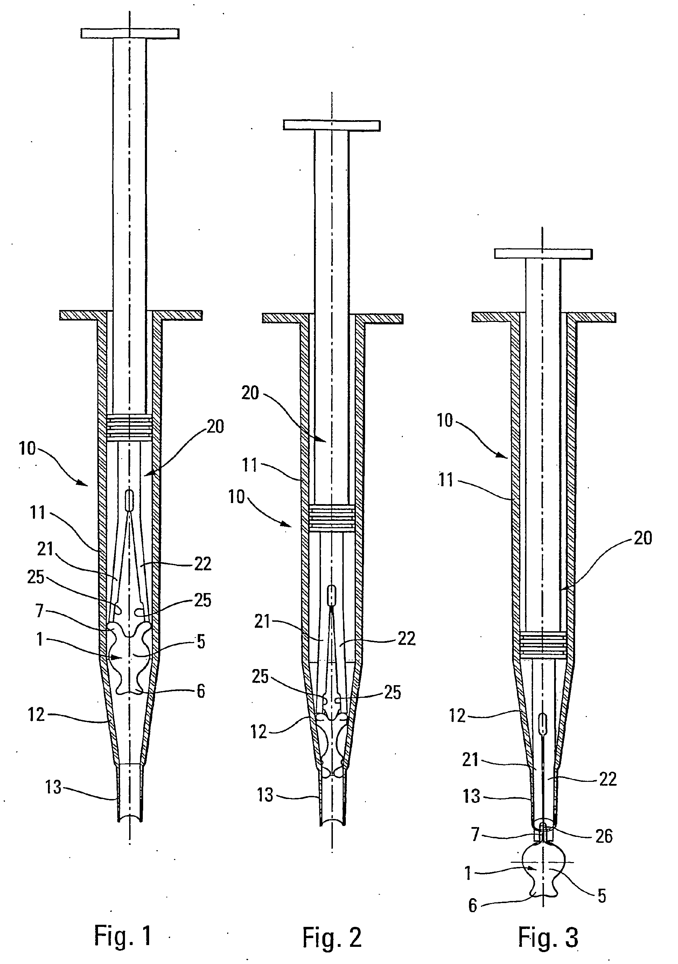

En référence aux figures 1 à 3, il est représenté un premier mode de réalisation de la présente invention. Dans ce premier mode de réalisation, la seringue comporte un corps 10, constitué d'une partie principale 11 cylindrique, d'une partie d'éjection 13 cylindrique, et de section transversale inférieure à ladite partie principale 11, et d'une partie conique 12 qui relie ladite partie principale 11 à ladite partie d'éjection 13. La partie principale 11 est adaptée à contenir la lentille 1 non déformée, comme représenté sur la figure 1. De préférence, la partie principale 11 est aplatie en section transversale, et est avantageusement réalisée avec un fond environ plat, comme cela est décrit dans le document WO 00/49974, qui est cité ici à titre de référence en ce qui concerne la forme du corps de seringue 10.

Le piston 20 comporte deux brins qui par flexibilité peuvent se rapprocher l'un de l'autre lorsque le piston coulisse dans la partie conique 12. La lentille intraoculaire 1 comporte une optique biconvexe dissymétrique 5 qui détermine le sens du pliage dès qu'elle est comprimée selon son diamètre. La lentille comporte une haptique inférieure 6, qui est celle disposée en aval dans le sens du déplacement de la lentille à l'intérieur du corps de seringue 10, et deux haptiques supérieures symétriques 7, qui coopèrent avec lesdits deux brins 21, 22 dudit piston 20.

Au départ, représenté sur la figure 1, la lentille est disposée de telle sorte que les deux brins du piston 21, 22 sont chacun en vis à vis d'une haptique supérieure 7. L'optique 5 est juste guidée lorsque le piston est actionné et l'haptique inférieure 6 qui est étroite est engagée sans contact dans la partie conique 12 de la seringue 10.

Lorsque l'on exerce une pression sur le piston 20, la lentille 1 est tout d'abord translatée sans déformation, jusqu'à ce que son diamètre optique transversal arrive au contact de la partie conique 12. En continuant la pression sur le piston 20, les haptiques supérieures 7 se déforment en premier, comme représenté schématiquement sur la figure 2, jusqu'à transmettre la poussée au bord de l'optique 5 et amorcer le pliage de celle-ci. En position intermédiaire, représentée sur la figure 2, les haptiques supérieures 7 restent déformées en accordéon, au contact avec l'extrémité des brins 21, 22. L'optique 5 en cours de pliage est presque roulée tout en étant en contact avec la paroi de la partie conique 12. L'haptique inférieure 6 a amorcé son pliage en suivant le mouvement de l'optique 5.

Après le pliage, la lentille 1 est poussée dans la partie d'éjection 13.

Selon l'invention, les deux brins 21, 22 du piston 20 comportent à leurs extrémités, en contact avec la lentille 1 et en particulier sur leurs côtés qui se font face, des évidements 25 qui définissent un profil à l'extrémité du piston 20. Ce profil est réalisé de telle sorte que lorsque ledit piston 20 atteint la partie d'éjection 13 de la seringue 10, il définit un espace 26 adapté à recevoir l'haptique supérieure 7 de la lentille, pour éviter un coincement de ladite lentille entre lesdits brins dudit piston en cours de distribution.

En fin de poussée, représentée sur la figure 3, l'optique 5 s'est redéployée à l'intérieur de l'oeil du patient, et les haptiques supérieures 7 sont plaquées l'une contre l'autre dans l'espace 26 prévu à cet effet. Avantageusement, comme cela est représenté sur la figure 3, les brins 21, 22 du piston 20 remplissent sensiblement la totalité de la section transversale de la partie d'éjection 13, en amont dudit espace 26. Plus particulièrement, en fin d'éjection de la lentille 1, ladite extrémité du piston 20 qui incorpore le profil 25 définissant l'espace 26 est disposé à l'extérieur du corps de seringue 10, et en particulier à l'extérieur de la partie d'éjection 13, de sorte que la totalité du volume de la partie d'éjection 13 est remplie par le piston 20.

Avantageusement, si les haptiques supérieures 7 peu comprimées ne se libèrent pas spontanément, une rétraction légère du piston 20 les dégage définitivement et la lentille intraoculaire 1 reprend sa forme initiale à l'intérieur de l'oeil du patient.

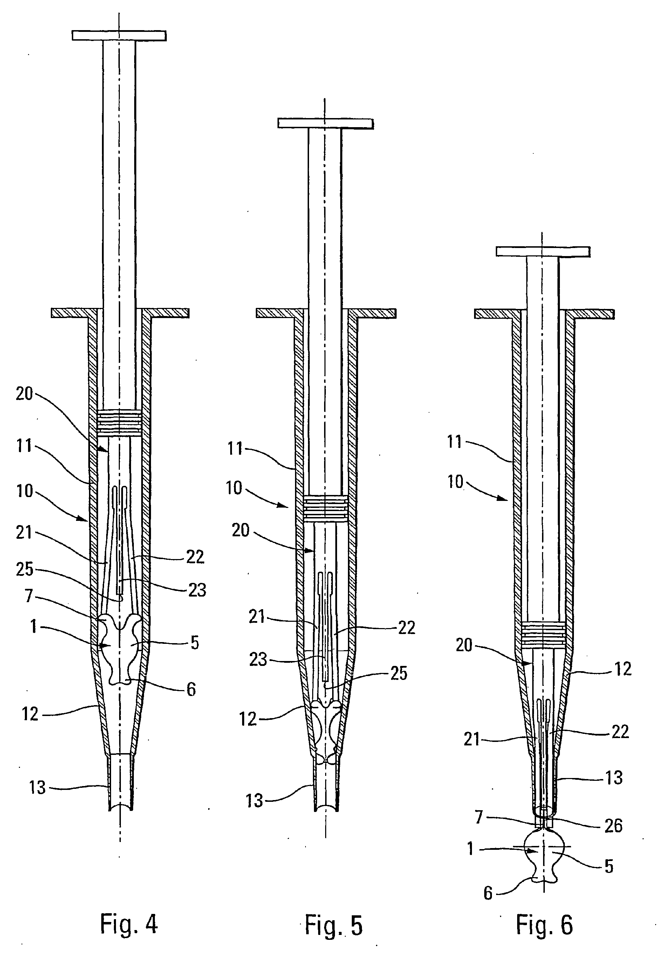

En référence aux figures 4 à 6, il est représenté un second mode de réalisation de la présente invention. Dans ce second mode de réalisation, le piston multibrins est constitué de deux brins latéraux 21, 22 de même longueur, et d'un brin central 23 qui est plus court que les deux brins latéraux 21, 22. Ce brin central 23 joue le rôle d'une entretoise qui, de par sa longueur raccourcie, définit un espace central 26 pour l'haptique supérieure 7 de la lentille 1, en fin de poussée. Le fonctionnement du dispositif est identique à celui discuté précédemment en référence aux figures 1 à 3.

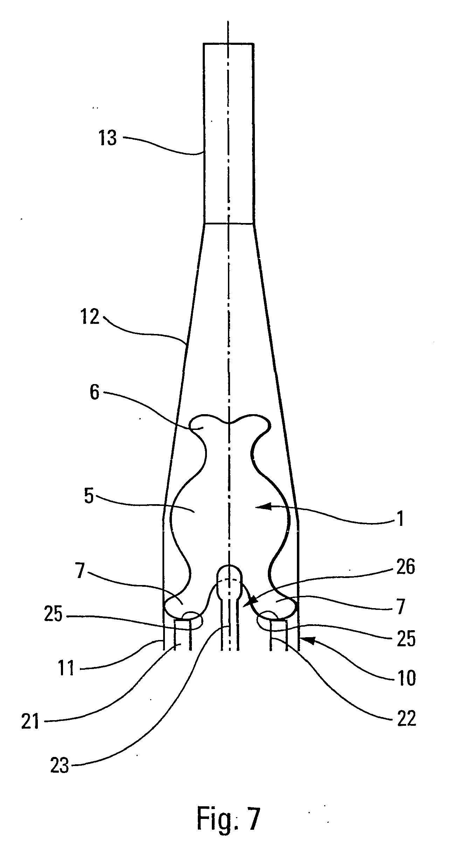

En référence à la figure 7, il est représenté un troisième mode de réalisation de la présente invention. Dans ce troisième mode de réalisation, le piston 20 comporte également deux brins latéraux 21, 22 de même longueur ainsi qu'un brin central 23 qui est plus long que les deux brins latéraux 21, 22. Ledit brin central 23 plus long est adapté à se placer entre les deux haptiques supérieures 7 de la lentille, et ledit brin central 23 définit donc un espace latéral 26 adapté à recevoir lesdites haptiques supérieures 7 de la lentille 1. Avantageusement, ledit brin central 23 est élargi et arrondi à son extrémité, afin d'améliorer son contact avec ladite lentille en vue de l'éjection de celle-ci.

Dans les trois modes de réalisation décrits ci-dessus, le piston 20 agit pour pousser la lentille et c'est la paroi ou les parois du corps de seringue 10 qui agissent avec ladite lentille pour la déformer. Plus précisément, ladite lentille 1 coulisse à l'intérieur du corps de seringue 10, et en particulier à l'intérieur de la partie conique 12 en étant poussée par les brins 21, 22 et éventuellement 23 du piston, le pliage étant obtenu par une conjonction de ladite poussée axiale du piston 20 et d'une sollicitation radiale exercée par la partie conique 12 du corps de seringue 10.

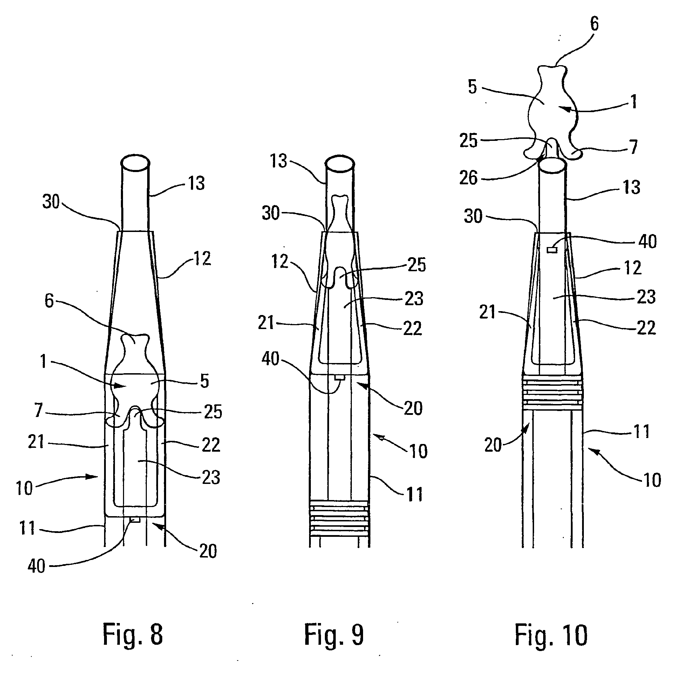

En référence aux figures 8 à 10, il va être décrit un quatrième mode de réalisation de l'invention. Dans ce quatrième mode de réalisation, la lentille 1 ne touche pas les parois du corps de seringue 10 jusqu'à ce que ladite lentille 1 atteigne la partie d'éjection 13 dudit corps de seringue. L'avantage de ce mode de réalisation est que ladite lentille n'est soumise à aucun frottement axial contre les parois du corps de seringue 10 pendant son pliage et son déplacement en direction de la partie d'éjection 13. A cet effet, le piston 20 est constitué de deux brins latéraux 21, 22, avantageusement de même longueur et formant une sorte de pince, et d'un brin central 23 qui est adapté à se déplacer par rapport auxdits deux brins latéraux 21, 22, notamment entre une position de déformation et une position d'éjection. Dans ce mode de réalisation, les deux brins latéraux 21, 22 sont avantageusement pourvus d'encoches afin de maintenir la lentille intraoculaire 1. Cette disposition a pour effet de supprimer tout contact direct entre l'optique 5 de la lentille 1 et la paroi interne du corps de seringue 10, tant que le pliage n'a pas été réalisé. Le frottement occasionné par la translation de la lentille 1 dans les précédents modes de réalisation est donc reporté entre les brins latéraux 21, 22 et la paroi interne de la seringue 10, notamment dans sa partie conique 12. Du point de vue de la lentille intraoculaire 1, l'effort subit lors du pliage est seulement radial, lorsque les brins 21, 22 se rapprochent l'un de l'autre en franchissant la partie conique 12, c'est à dire que tout se passe comme si la lentille 1 était resserrée entre les deux mors d'une pince de pliage conventionnelle.

Dans ce quatrième mode de réalisation, le piston comprend un brin central 23 qui est solidaire desdits deux brins latéraux 21, 22 formant la pince jusqu'à ce que la lentille 1 soit pliée c'est à dire jusqu'à ce que la lentille atteigne la partie d'éjection 13. A ce niveau là, le brin central 23 peut se désolidariser desdits brins latéraux 21, 22, afin d'assurer l'éjection de ladite lentille à travers ladite partie d'éjection 13. Plus particulièrement, une butée 30 est prévue, avantageusement au niveau de la jonction de la partie conique 12 et de la partie d'éjection 13, ladite butée 30 bloquant le déplacement desdits brins latéraux 21, 22, ledit blocage provoquant la libération ou la désolidarisation du brin central 23 adapté à éjecter ladite lentille 1. Ainsi, lors de la phase finale de l'éjection, les brins latéraux 21, 22 qui ne peuvent pas pénétrer dans l'embout d'injection 13 sont maintenus en butée. Simultanément, le brin central 23, qui peut par exemple être réalisé par un brin central unique ou par un ensemble de brins centraux, continu sa course pour propulser la lentille intraoculaire à travers l'embout ou partie d'éjection 13. Le dispositif comporte donc un système permettant de solidariser puis de désolidariser les brins latéraux 21, 22 formant pinces avec le brin central 23 assurant l'éjection de la lentille 1 en fin de poussée.

De manière évidente, différentes solutions techniques peuvent être imaginées pour réaliser ceci, comme par exemple des doubles pistons coaxiaux, une vis interne mobilisant uniquement le brin central lorsque ledit piston atteint ladite partie d'éjection 13, ou une liaison entre les brins latéraux 21, 22 et le brin central 23 avec un élément de rupture ou point dur qui ne solidarise ces éléments que jusqu'à une certaine valeur de l'effort de poussée. C'est cet exemple de réalisation qui est représenté sur les figures, qui montrent un bossage 40 réalisé sur le brin central 23 qui solidarise ledit brin central avec lesdits brins latéraux 21, 22 jusqu'à ce que ceux-ci arrivent en butée contre la butée 30 au niveau de la jonction entre la partie conique 12 et la partie d'éjection 13. Lorsque lesdits brins latéraux 21, 22 arrivent contre cette butée 30, une continuation de la poussée sur le piston 20augment la pression exercée sur le brin central 23, qui fait se déformer la partie coopérant avec le bossage 40 de sorte que ledit brin central 23 peut se désolidariser desdits brins latéraux 21, 22 pour assurer l'éjection de ladite lentille.

Selon l'invention, dans ce quatrième mode de réalisation, le brin central 23 comporte une extrémité 25 qui définit un espace 26 adapté à recevoir la ou les haptique(s) supérieure(s) 7 de la lentille 1. Dans cet exemple de réalisation comme représenté sur les dessins, ledit profil 25 est réalisé par une pointe de diamètre inférieur qui vient se placer entre les deux haptiques supérieures 7. Bien évidemment, on pourrait envisager un profil différent, par exemple un profil 25 définissant un espace central, qui serait particulièrement adapté pour des lentilles comportant une seule haptique supérieure 7.

Ce quatrième mode de réalisation présente les avantages suivants :
- en fin de translation dans la partie cylindrique 11 du corps de seringue 10, la lentille 1 est juste maintenue dans la pince formée par les brins latéraux 21, 22, sans être déformée ;
- après passage dans la partie conique 12, les brins latéraux 21, 22 sont complètement fermés et la lentille intraoculaire 1 est pliée et engagée dans l'axe de la partie d'éjection 13 formant embout de la seringue. Les deux brins latéraux 21, 22 sont arrivés en butée contre la butée 30, avantageusement réalisés sous la forme de deux rampes leur servant de guide dans la partie conique. Les deux petits bossages 40 assurant le rôle de point dur n'ont pas encore franchi l'entretoise reliant les deux brins latéraux 21, 22, et qui solidarise ceux-ci avec ledit brin central 23 ;
- en fin de poussée, la lentille 1 a été éjectée et s'est redéployée, et l'extrémité du brin central 23 (qui est unique dans l'exemple représenté et qui définit deux espaces latéraux 26 de part et d'autre de son extrémité en pointe 25) a complètement débouché hors de l'orifice de la partie d'éjection 13.

La présente invention a été décrite ci-dessus en référence à quatre modes de réalisation avantageux de celle-ci, mais il est clair qu'elle ne se limite pas à ces modes de réalisation. En particulier, la forme des haptiques peut être quelconque, et ces haptiques ne sont pas nécessairement réalisées de manière monobloc avec l'optique 5 de la lentille. Toutefois, une haptique inférieure 6 étroite coopère avantageusement avec la conicité de la partie conique 12 de la seringue. De même, la forme des haptiques supérieures 7 est particulièrement adaptée à la transmission de la poussée par ledit piston multibrins, en particulier par l'intermédiaire desdits brins latéraux dudit piston. D'autre part, des lentilles comportant des haptiques à anses rapportées ou des lentilles monoblocs ayant des formes différentes, par exemples des anses en forme de C, des navettes, des haptiques à quatre points d'appui, ou similaires sont également couvertes par la présente invention.

D'autres modifications peuvent être apportées par l'homme du métier sans sortir du cadre de la présente invention, telle que définie par les revendications annexées.

## Revendications

1. Ensemble comprenant un injecteur et une lentille intraoculaire, ladite lentille intraoculaire (1) comportant une optique (5), au moins une haptique inférieure (6), et au moins une haptique supérieure (7), ledit injecteur comprenant un corps de seringue (10) comportant une partie principale (11), de préférence cylindrique, une partie d'éjection (13), de préférence cylindrique et de section transversale inférieure à celle de la partie principale (11), et une partie conique (12) reliant ladite partie principale (11) à ladite partie d'éjection (13), un piston (20) étant adapté à se déplacer à l'intérieur dudit corps de seringue (10) pour déplacer ladite lentille (1) de la partie principale (11) vers la partie d'éjection (13), puis pour éjecter ladite lentille (1) hors de ladite partie d'éjection (13), ledit piston (20) comportant au moins deux brins (21, 22) pouvant se rapprocher l'un de l'autre lorsque le piston (20) coulisse dans ladite partie conique (12), **caractérisé en ce que** ledit piston (20), lorsqu'il atteint la partie d'éjection (13), comporte à son extrémité en contact avec la lentille (1) un profil (25) définissant au moins un espace (26) adapté à recevoir ladite au moins une haptique supérieure (7) de ladite lentille (1).

2. Ensemble selon la revendication 1, dans lequel, en amont dudit espace (26), ledit piston (20) remplit sensiblement toute la section transversale de ladite partie d'éjection (13).

3. Ensemble selon la revendication 1 ou 2, dans lequel ledit espace (26) est central et est défini entre lesdits aux moins deux brins (21, 22) du piston (20).

4. Ensemble selon l'une quelconque des revendications 1 à 3, dans lequel ledit piston (20) est constitué de deux brins latéraux (21, 22) comportant à leurs extrémités en contact avec la lentille (1) un évidement (25) sur leurs côtés se faisant face, de sorte que lorsque lesdits deux brins (21, 22) sont sollicités l'un contre l'autre dans ladite partie d'éjection (13), lesdits évidements (25) définissent un espace central (26).

5. Ensemble selon l'une quelconque des revendications 1 à 3, dans lequel ledit piston (20) est constitué de deux brins latéraux de même longueur (21, 22) et d'un brin central (23) plus court, de sorte que lorsque lesdits brins latéraux (21, 22) sont sollicités contre ledit brin central (23) dans ladite partie d'éjection (13), ledit brin central plus court (23) définit un espace central (26) entre les extrémités desdits brins latéraux (21, 22).

6. Ensemble selon la revendication 1 ou 2, dans lequel ledit piston (20) est constitué de deux brins latéraux de même longueur (21, 22) et d'un brin central (23) plus long, de sorte que lorsque lesdits brins latéraux (21, 22) sont sollicités contre ledit brin central (23) dans ladite partie d'éjection (13), ledit brin central plus long (23) définit un espace latéral (26) autour de l'extrémité plus longue (25) dudit brin central (23).

7. Ensemble selon l'une quelconque des revendications précédentes, dans lequel lesdits brins (21, 22, 23) du piston (20) sont adaptés à pousser la lentille (1) dans le corps de seringue (10), la lentille (1) glissant contre les parois (11, 12, 13) dudit corps de seringue (10) et étant déformée par ladite partie conique (12).

8. Ensemble selon la revendication 1 ou 2, dans lequel ledit piston comporte deux brins latéraux (21, 22) et un brin central (23) adapté à se déplacer par rapport auxdits brins latéraux (21, 22) entre une position de déformation et une position d'éjection.

9. Ensemble selon la revendication 8, dans lequel ladite lentille (1) est disposée entre lesdits brins latéraux (21, 22) formant une pince, de telle sorte que ladite lentille ne glisse pas contre les parois (11, 12, 13) dudit corps de seringue (10) pendant sa déformation, ladite déformation étant réalisée par lesdits brins latéraux (21, 22) qui se rapprochent lorsque le piston (20) coulisse dans ladite partie conique (12) du corps de seringue (10).

10. Ensemble selon la revendication 8 ou 9, dans lequel ledit brin central (23) se déplace ensemble avec lesdits brins latéraux (21, 22) pendant la déformation de ladite lentille (1), jusqu'à une butée (30) bloquant lesdits brins latéraux (21, 22), ledit brin central (23) se désolidarisant alors desdits brins latéraux (21, 22) pour éjecter ladite lentille déformée (1).

11. Ensemble selon la revendication 10, dans lequel ladite butée (30) est formée au niveau de la jonction entre la partie conique (12) et la partie d'éjection (13) du corps de seringue (10).

12. Ensemble selon l'une quelconque des revendications 8 à 11, dans lequel ledit brin central (23) comporte à son extrémité en contact avec la lentille (1), un profil (25) définissant un espace central ou latéral (26) pour recevoir ladite au moins une haptique supérieure de ladite lentille (1).

13. Ensemble selon l'une quelconque des revendications 10 à 12, dans lequel ledit brin central (23) est relié auxdits brins latéraux (21, 22) par un mécanisme de liaison mécanique (40) qui désolidarise automatiquement ledit brin central (23) desdits brins latéraux (21, 22) lorsque ceux-ci atteignent ladite butée (30).

14. Ensemble selon l'une quelconque des revendications précédentes, dans lequel, en fin d'éjection de la lentille (1), ledit profil d'extrémité (25) du piston (20), qui définit ledit espace (26), est disposé à l'extérieur du corps de seringue (10), en particulier à l'extérieur de ladite partie d'éjection (13).

15. Ensemble selon l'une quelconque des revendications précédentes, dans lequel ladite partie principale (11) du corps de seringue (10) est adaptée à contenir la lentille (1) non déformée.

16. Ensemble selon l'une quelconque des revendications précédentes, dans lequel ladite partie principale (11) du corps de seringue (10) est aplatie en section transversale.

## Claims

1. An assembly comprising an injector and an intraocular lens, said intraocular lens (1) being made up of an optic (5), of at least one lower haptic (6), and of at least one upper haptic (7), said injector comprising a syringe body (10) having a preferably cylindrical main portion (11), a preferably cylindrical discharge portion (13) that is preferably of smaller cross-section than the cross-section of the main portion (11), and a conical portion (12) connecting said main portion (11) to said discharge portion (13), a plunger (20) being adapted to move inside said syringe body (10) so as move said lens (1) from the main portion (11) to the discharge portion (13), and then to discharge said lens (1) out of said discharge portion (13), said plunger (20) being provided with at least two strands (21, 22) that can move towards each other while the plunger (20) is sliding inside said conical portion (12), said assembly being **characterized in that**, when said plunger (20) reaches the discharge portion (13) said plunger has a profile (25) at its end in contact with the lens (1), said profile defining at least one space (26) adapted to receive said at least one upper haptic (7) of said lens (1).

2. An assembly according to claim 1, in which, upstream from said space (26), said plunger (20) fills substantially the entire cross section of said discharge portion (13).

3. An assembly according to claim 1 or claim 2, in which said space (26) is central and is defined between said at least two strands (21, 22) of the plunger (20).

4. An assembly according to any one of claims 1 to 3, in which said plunger (20) is made up of two side strands (21, 22), each of which is provided with a recess (25) on its end that is contact with the lens (1), the recesses being provided in those sides of the side strands that face each other, so that, when said two strands (21, 22) are urged together inside said discharge portion (13), said recesses (25) define a central space (26).

5. An assembly according to any one of claims 1 to 3, in which said plunger (20) is made up of two same-length side strands (21, 22) and of a shorter central strand (23), so that, when said side strands (21, 22) are urged against said central strand (23) inside said discharge portion (13), said shorter central portion (23) defines a central space (26) between the ends of said side strands (21, 22).

6. An assembly according to claim 1 or 2, in which said plunger (20) is made up of two same-length side strands (21, 22) and of a longer central strand (23), so that, when said side strands (21, 22) are urged against said central strand (23) inside said discharge portion (13), said longer central strand (23) defines a side space (26) around the longer end (25) of said central strand (23).

7. An assembly according to any preceding claim, in which said strands (21, 22, 23) of the plunger (20) are adapted to push the lens (1) into the syringe body (10), the lens (1) sliding against the walls (11, 12, 13) of said syringe body (10) and being deformed by said conical portion (12).

8. An assembly according to claim 1 or 2, in which said plunger is made up of two side strands (21, 22) and of a central strand (23) adapted to move relative to said side strands (21, 22) between a deforming position and a discharging position.

9. An assembly according to claim 8, in which said lens (1) is disposed between said side strands (21, 22) which form a pair of tweezers, so that said lens does not slide against the walls (11, 12, 13) of said syringe body (10) while said lens is being deformed, said deformation being implemented by said side strands (21, 22) which move towards each other while the plunger (20) is sliding inside said conical portion (12) of the syringe body (10).

10. An assembly according to claim 8 or 9, in which said central strand (23) moves with said side strands (21, 22) while said lens (1) is being deformed, until an abutment (30) stops said side strands (21, 22), said central strand (23) then being released from said side strands (21, 22) so as to discharge said deformed lens (1).

11. An assembly according to claim 10, in which said abutment (30) is formed where the conical portion (12) meets the discharge portion (13) of the syringe body (10).

12. An assembly according to any one of claims 8 to 11, in which, at its end in contact with the lens (1), said central strand (23) has a profile (25) defining a central or side space (26) for receiving said at least one upper haptic of said lens (1).

13. An assembly according to any one of claims 10 to 12, in which said central strand (23) is connected to said side strands (21, 22) via a mechanical link mechanism (40) which automatically releases said central strand (23) from said side strands (21, 22) when said side strands reach said abutment (30).

14. An assembly according to any preceding claim, in which, at the end of discharge of the lens (1), said end profile (25) of the plunger (20), which profile defines said space (26), is disposed outside the syringe body (10), in particular outside said discharge portion (13).

15. An assembly according to any preceding claim, in which said main portion (11) of the syringe body (10) is adapted to containing the lens (1) in the non-deformed state.

16. An assembly according to any preceding claim, in which said main portion (11) of the syringe body (10) is oblate in cross-section.

## Patentansprüche

1. Ensemble, das einen Injektor und eine Intraokular-Linse umfasst, wobei die Intraokular-Linse (1) eine Optik (5), wenigstens einen unteren Fixierteil (6) und wenigstens einen oberen Fixierteil (7) aufweist, und wobei der Injektor einen Spritzenkörper (10) besitzt, der einen vorzugsweise zylindrischen Hauptteil (11), einen vorzugsweise zylindrischen Ausstoßteil (13) mit einem Querschnitt, der kleiner ist als der des Hauptteils (11), und einen konischen Teil (12) umfasst, der den Hauptteil (11) mit dem Ausstoßteil (1,3) verbindet, sowie einen Kolben (20), der sich im Inneren des Spritzenkörpers (10) verschieben kann, um die Linse (1) aus dem Hauptteil (11) zum Ausstoßteil (13) zu verschieben und die Linse (1) dann aus dem Ausstoßteil (13) hinaus auszustoßen, wobei der Kolben (20) wenigstens zwei Stiele (21, 22) umfasst, die sich aneinander annähern können, wenn der Kolben (20) in dem konischen Teil (12) gleitet, **dadurch gekennzeichnet, dass** der Kolben (20) dann, wenn er den Ausstoßteil (13) erreicht, an seinem Ende in Berührung mit der Linse (1) ein Profil (25) aufweist, das wenigstens einen Freiraum (26) definiert, der den wenigstens einen oberen Fixierteil (7) der Linse (1) aufnehmen kann.

2. Ensemble nach Anspruch 1, bei dem stromaufwärts von besagtem Freiraum (26) der Kolben (20) im Wesentlichen den gesamten Querschnitt des Ausstoßteils (13) ausfüllt.

3. Ensemble nach Anspruch 1 oder 2, bei dem der Freiraum (26) zentral angeordnet und zwischen den wenigstens zwei Stielen (21, 22) des Kolbens (20) ausgebildet ist.

4. Ensemble nach einem der Ansprüche 1 bis 3, bei dem der Kolben (20) von zwei seitlichen Stielen (21, 22) gebildet ist, die an ihren mit der Linse (1) in Berührung stehenden Enden an ihren aufeinander zu weisenden Seiten eine Ausnehmung (25) derart aufweisen, dass dann, wenn die beiden. Stiele (21, 22) in dem Ausstoßteil (13) gegeneinander gedrückt werden, diese Ausnehmungen (25) einen zentralen Freiraum (26) bilden.

5. Ensemble nach einem der Ansprüche 1 bis 3, bei dem der Kolben (20) von zwei seitlichen Stielen (21, 22) gleicher Länge und einem kürzeren zentralen Stiel (23) derart gebildet wird, dass dann, wenn die seitlichen Stiele (21, 22) gegen den zentralen Stiel (23) im Ausstoßteil (13) vorgespannt werden, der kürzere zentrale Stiel (23) einen zentralen Freiraum (26) zwischen den Enden der seitlichen Stiele (21, 22) bildet.

6. Ensemble nach Anspruch 1 oder 2, bei dem der Kolben (20) von zwei seitlichen Stielen (21, 22) mit gleicher Länge und einem längeren zentralen Stiel (23) derart gebildet wird, dass dann, wenn die seitlichen Stiele (21, 22) im Ausstoßteil (13) gegen den zentralen Stiel (23) vorgespannt werden, der längere zentrale Stiel (23) einen seitlichen Freiraum (26) um das längere Ende (25) des zentralen Stiels (23) herum bildet.

7. Ensemble nach einem der vorhergehenden Ansprüche, bei dem die Stiele (21, 22, 23) des Kolbens (20) geeignet sind, die Linse (1) im Spritzenkörper (10) zu schieben, wobei die Linse (1) an den Wänden (11, 12, 13) des Spritzenkörpers (10) gleitet und durch den konischen Teil (12) verformt wird.

8. Ensemble nach Anspruch 1 oder 2, bei dem der Kolben (2) seitliche Stiele (21, 22) und einen zentralen Stiel (23) umfasst, der sich bezüglich der seitlichen Stiele (21, 22) zwischen einer Verformungsstellung und einer Ausstoßstellung verschieben kann.

9. Ensemble nach Anspruch 8, bei dem die Linse (1) zwischen den seitlichen Stielen (21, 22) angeordnet ist, die eine Klemme derart bilden, dass die Linse nicht an den Wänden (11, 12, 13) des Spritzenkörpers (10) während ihrer Verformung gleitet, wobei diese Verformung von den seitlichen Stielen (21, 22) durchgeführt wird, die sich aneinander annähern, während der Kolben (20) in dem konischen Teil (12) des Spritzenkörpers (10) gleitet.

10. Ensemble nach Anspruch 8 oder 9, bei dem der zentrale Stiel (23) sich zusammen mit den seitlichen Stielen (21, 22) während der Verformung der Linse (1) verschiebt, bis zu einem Anschlag (30), der die seitlichen Stiele (21, 22) blockiert, worauf sich der zentrale Stiel (23) von den seitlichen Stielen (21, 22) trennt, um die verformte Linse (1) auszustoßen.

11. Ensemble nach Anspruch 10, bei dem der Anschlag (30) im Bereich der Verbindung zwischen dem konischen Teil (12) und dem Ausstoßteil (13) des Spritzenkörpers (10) ausgebildet ist.

12. Ensemble nach einem der Ansprüche 8 bis 11, bei dem der zentrale Stiel (23) an seinem mit der Linse (1)in Berührung stehenden Ende ein Profil (25) aufweist, das einen zentralen oder seitlichen Freiraum (26) bildet, um den wenigstens einen oberen Fixierteil der Linse (1) aufzunehmen.

13. Ensemble nach einem der Ansprüche 10 bis 12, bei dem der zentrale Stiel (23) mit den seitlichen Stielen (21, 22) durch einen mechanischen Verbindungsmechanismus (40) verbunden ist, der automatisch den zentralen Stiel (23) von den seitlichen Stielen (21, 22) trennt, wenn diese den Anschlag (30) erreichen.

14. Ensemble nach einem der vorhergehenden Ansprüche, bei dem am Ende des Ausstoßes der Linse (1) das Endprofil (25) des Kolbens (20), das den Freiraum (26) definiert, außerhalb des Spritzenkörpers (10), insbesondere außerhalb des Ausstoßteils (13) angeordnet ist.

15. Ensemble nach einem der vorhergehenden Ansprüche, bei dem der Hauptteil (11) des Spritzenkörpers (10) geeignet ist, die nicht verformte Linse (1) aufzunehmen.

16. Ensemble nach einem der vorhergehenden Ansprüche, bei dem der Hauptteil (11) des Spritzenkörpers (10) einen abgeflachten Querschnitt aufweist.
